# FASCICULE DE BREVET EUROPEEN

(11) **EP 2 151 430 B1**
(45) Date de publication et mention de la délivrance du brevet: **03.08.2011**
(21) Numéro de dépôt: 09290608.0
(22) Date de dépôt: 04.08.2009
(51) Int. Cl.: C07C 231/24, C07C 233/18

(54) **NOUVEAU PROCÉDÉ D'OBTENTION DE LA FORME CRISTALLINE V DE L'AGOMÉLATINE**
NEUES HERSTELLUNGSVERFAHREN DER KRISTALLINEN FORM V VON AGOMELATIN
NEW METHOD FOR OBTAINING THE CRYSTALLINE FORM V OF AGOMELATINE

(30) Priorité: 05.08.2008 FR 0804466
(43) Date de publication de la demande: 10.02.2010
(73) Titulaire: Les Laboratoires Servier, 92284 Suresnes Cedex (FR)
(72) Inventeur: Martins, Damien, 76000 Rouen (FR); Coquerel, Gérard, 76520 Boos (FR); Linol, Julie, 76100 Rouen (FR); Langlois, Pascal, 76210 Saint Jean De La Neuville (FR)

(56) Documents cités:
- EP-A- 1 564 202
- EP-A- 1 752 443
- TINANT B ET AL: "N-[2-(7-METHOXY-1-NAPHTHYL)ETHYL]ACETAMID E, A POTENT MELATONIN ANALOG" ACTA CRYSTALLOGRAPHICA SECTION C. CRYSTAL STRUCTURECOMMUNICATIONS, MUNKSGAARD, COPENHAGEN, DK, vol. C50, no. 6, 1 janvier 1994 (1994-01-01), pages 907-910, XP009047983 ISSN: 0108-2701

## Description

La présente invention concerne un nouveau procédé d'obtention de la forme cristalline V de l'agomélatine ou *N*-[2-(7-méthoxy-1-naphtyl)éthyl]acétamide de formule (I) :

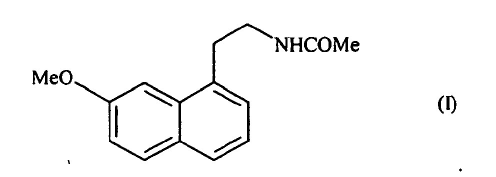

L'agomélatine ou *N*-[2-(7-méthoxy-1-naphtyl)éthyl]acétamide possède des propriétés pharmacologiques intéressantes.

Il présente en effet la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans le brevet européen EP 0 447 285.

Compte tenu de l'intérêt pharmaceutique de ce composé, il était primordial de l'obtenir avec une excellente pureté, et notamment sous une forme parfaitement reproductible, présentant des caractéristiques intéressantes permettant son stockage prolongé sans conditions particulières de température, de lumière, d'humidité ou de taux d'oxygène.

La demande de brevet EP 1 752 443 décrit une forme cristalline bien définie de l'agomélatine, la forme cristalline V caractérisée par son diagramme de diffraction X sur poudre suivant, mesuré sur un diffractomètre Siemens D5005 (anticathode de cuivre) et exprimé en termes de distance inter-réticulaire d, d'angle de Bragg 2 thêta, et d'intensité relative (exprimée en pourcentage par rapport à la raie la plus intense) :

| **2-Theta (°) exp.** | **d (Å) exp.** | **Intensité (%)** |
|---|---|---|
| 9,84 | 8,979 | 17 |
| 12,40 | 7,134 | 15 |
| 13,31 | 6,646 | 19 |
| 15,14 | 5,848 | 18 |
| 15,98 | 5,543 | 18 |
| 16,62 | 5,329 | 19 |
| 17,95 | 4,939 | 100 |
| 18,88 | 4,697 | 65 |
| 20,49 | 4,332 | 24 |
| 20,99 | 4,228 | 34 |
| 23,07 | 3,852 | 39 |
| 23,44 | 3,792 | 36 |
| 24,28 | 3,663 | 58 |
| 25,10 | 3,545 | 19 |
| 26,02 | 3,422 | 15 |
| 26,82 | 3,322 | 19 |
| 27,51 | 3,239 | 16 |

Cette forme cristalline, parfaitement définie, obtenue de façon reproductible, présente des propriétés morphologiques tout à fait intéressantes, avec notamment une surface spécifique bien plus grande que pour les autres formes décrites. Néanmoins, elle présente une tenue dans le temps un peu faible, et dans tous les cas inférieure à 6 mois.

La demanderesse a présentement mis au point un nouveau procédé d'obtention de l'agomélatine sous la forme cristalline V, de façon parfaitement reproductible, permettant d'augmenter sa tenue dans le temps. Ce nouveau procédé permet ainsi d'obtenir l'agomélatine sous la forme cristalline V avec des propriétés compatibles avec son usage pharmaceutique.

La forme V n'a pu être obtenue que par broyage dit « de haute énergie » ou par ensemencement à partir de cette forme structuralement pure obtenue par broyage. La demanderesse a maintenant découvert que, de façon surprenante, il était possible d'obtenir cette forme par atomisation. En effet, l'atomisation est une technique couramment utilisée pour accéder à des particules solides de petites tailles. Souvent le matériau résultant est amorphe (Amorphous state, Polymorphism in pharmaceutical industry, Ed. R. Hilfiker, Wiley-VCH Weinheim 2006, Chapitre X, p.259-285, S. Petit et G. Coquerel). Au contraire, dans la présente invention, l'atomisation a permis d'obtenir une forme cristalline bien définie, la forme V, mais qui de plus présente une tenue dans le temps bien supérieure.

Plus spécifiquement, la présente invention concerne le nouveau procédé d'obtention de l'agomélatine de formule (I) sous la forme cristalline V, caractérisé en ce qu'une solution d'agomélatine dissoute dans un ou deux solvants miscibles en toutes proportions et dont la température d'ébullition est inférieure à 120°C, est atomisée dans un atomiseur.

L'atomisation est une technique communément utilisée dans les milieux agroalimentaires et pharmaceutiques pour sécher une solution pulvérisée à travers un gaz chaud. En pratique, le gaz utilisé pour sécher la solution est de l'air mais certaines productions pharmaceutiques utilisant des solvants organiques nécessitent un gaz inerte comme gaz asséchant évitant ainsi certaines dégradations.

Les opérations de cristallisation selon la présente invention sont réalisées préférentiellement au moyen d'un atomiseur (Spray Dryer). Encore plus préférentiellement, l'atomisation selon l'invention est réalisée suivant le principe de l'atomisation par buse en courant parallèle, et plus préférentiellement en co-courant c'est-à-dire que la solution pulvérisée et le gaz de dessication s'écoulent dans la même direction.

De façon avantageuse, le gaz utilisé est l'air comprimé ou un gaz inerte comme l'azote par exemple.

Les solvants préférés du procédé selon l'invention sont l'éthanol, l'eau, l'éther isopropylique, le méthanol, l'acétate d'éthyle ou l'acétone.

La concentration de la solution d'agomélatine utilisée est au minimum de 5 g/l et plus préférentiellement on utilise une solution à 10 g/l.

Avantageusement, la température d'entrée du procédé selon l'invention est comprise entre 70°C et 120°C.

Dans le procédé de cristallisation selon l'invention, on peut utiliser l'agomélatine de formule (I) obtenu par n'importe quel procédé.

Les exemples ci-dessous illustrent l'invention, mais ne la limitent en aucune façon.

### Exemple 1 : Forme cristalline V du N-[2-(7-Méthoxy-1-naphtyl)éthyl]acétamide

Une solution à 10 g/l d'agomélatine dans un mélange éthanol/éther isopropylique (50/50 : v/v) est introduite dans un atomiseur de type Mini Spray Dryer BUCHI 190. La température d'entrée de la chambre de séchage est égale à 90°C, et la température de sortie est de 66°C. La poudre atomisée est récupérée dans le bol collecteur et a été caractérisée par les données cristallographiques suivantes :
1) diagramme obtenu sur le diffractomètre D5005 de Siemens avec un domaine angulaire 3°- 30° en 20, un pas de 0,04° et 4 s par pas :
   - maille cristalline monoclinique,
   - paramètres de maille : a = 11,967 Å, b = 17,902 Å, c = 15,423 Å, β = 124,5°
   - groupe d'espace : P2₁/n
   - nombre de molécules dans la maille : 8 (Z'=2)
   - volume de la maille : Vₘₐᵢₗₗₑ = 2720,0 Å³
2) diagramme de diffraction X sur poudre suivant, mesuré sur un diffractomètre Siemens D5005 (anticathode de cuivre) et exprimé en termes de distances inter-réticulaires d, d'angles de Bragg 2 thêta, et d'intensités relatives (exprimées en pourcentage par rapport à la raie la plus intense) :

| **2-Theta (°) exp.** | **d (Å) exp.** | **Intensité (%)** | **2-Theta (°) calc.** |
|---|---|---|---|
| 9,84 | 8,979 | 17 | 9,85 |
| 12,40 | 7,134 | 15 | 12,46 |
| 13,31 | 6,646 | 19 | 13,33 |
| 15,14 | 5,848 | 18 | 15,16 |
| 15,98 | 5,543 | 18 | 15,91 |
| 16,62 | 5,329 | 19 | 16,66 |
| 17,95 | 4,939 | 100 | 17,96 |
| 18,88 | 4,697 | 65 | 18,93 |
| 20,49 | 4,332 | 24 | 20,52 |
| 20,99 | 4,228 | 34 | 20,99 |
| 23,07 | 3,852 | 39 | 23,11 |
| 23,44 | 3,792 | 36 | 23,48 |
| 24,28 | 3,663 | 58 | 24,27 |
| 25,10 | 3,545 | 19 | 25,18 |
| 26,02 | 3,422 | 15 | 26,02 |
| 26,82 | 3,322 | 19 | 26,85 |
| 27,51 | 3,239 | 16 | 27,56 |

### Exemple 2 : Tenue dans le temps de la forme cristalline V du N-[2-(7-Méthoxy-1-naphtyl)éthyl]acétamide obtenue par atomisation

Un échantillon de 1 g du composé obtenu dans l'Exemple 1 a été placé dans des conditions de stockage classiques : pression et température ambiantes. Après 21 mois, le diffractogramme de l'échantillon obtenu n'a pas évolué, et reste caractéristique de la forme V obtenue.

## Revendications

1. Procédé d'obtention de la forme cristalline V de l'agomélatine de formule (I) : 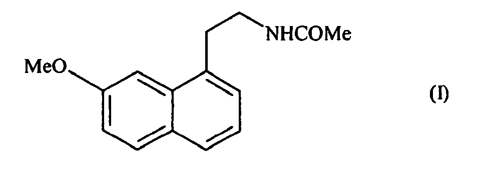 **caractérisé en ce qu'**une solution d'agomélatine dissout dans un ou deux solvants miscibles en toutes proportions et dont la température d'ébullition est inférieure à 120°C, est atomisée dans un atomiseur.

2. Procédé de préparation de la forme cristalline V du composé de formule (I) selon la revendication 1, **caractérisé en ce que** le composé obtenu présente le diagramme de diffraction X sur poudre suivant, mesuré sur un diffractomètre Siemens D5005 (anticathode de cuivre) et exprimé en termes de distances inter-réticulaires d, d'angles de Bragg 2 thêta, et d'intensités relatives (exprimées en pourcentage par rapport à la raie la plus intense) :
| **2-Theta (°) exp.** | **d (Å) exp.** | **Intensité (%)** | **2-Theta (°) calc.** |
|---|---|---|---|
| 9,84 | 8,979 | 17 | 9,85 |
| 12,40 | 7,134 | 15 | 12,46 |
| 13,31 | 6,646 | 19 | 13,33 |
| 15,14 | 5,848 | 18 | 15,16 |
| 15,98 | 5,543 | 18 | 15,91 |
| 16,62 | 5,329 | 19 | 16,66 |
| 17,95 | 4,939 | 100 | 17,96 |
| 18,88 | 4,697 | 65 | 18,93 |
| 20,49 | 4,332 | 24 | 20,52 |
| 20,99 | 4,228 | 34 | 20,99 |
| 23,07 | 3,852 | 39 | 23,11 |
| 23,44 | 3,792 | 36 | 23,48 |
| 24,28 | 3,663 | 58 | 24,27 |
| 25,10 | 3,545 | 19 | 25,18 |
| 26,02 | 3,422 | 15 | 26,02 |
| 26,82 | 3,322 | 19 | 26,85 |
| 27,51 | 3,239 | 16 | 27,56 |

3. Procédé de préparation de la forme cristalline V du composé de formule (I) selon la revendication 1, **caractérisé en ce que** les solvants utilisés sont l'éthanol et l'éther isopropylique.

4. Procédé de préparation de la forme cristalline V du composé de formule (I) selon la revendication 1, **caractérisé en ce que** le gaz utilisé est l'azote.

5. Procédé de préparation de la forme cristalline V du composé de formule (I) selon la revendication 1, **caractérisé en ce que** la concentration de la solution d'agomélatine utilisée est au minimum de 5 g/l.

## Claims

1. Process for obtaining the crystalline form V of agomelatine of formula (I): 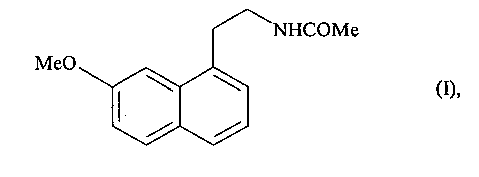 **characterised in that** a solution of agomelatine that has been dissolved in one or two solvents which are miscible in any proportion and whose boiling point is less than 120°C is atomised in an atomiser.

2. Process for the preparation of the crystalline form V of the compound of formula (I) according to claim 1, **characterised in that** the compound obtained has the following X-ray powder diffraction diagram, measured using a Siemens D5005 diffractometer (copper anticathode) and expressed in terms of interplanar distances d, Bragg's angles 2 theta, and relative intensities (expressed as a percentage in relation to the most intense line):
| **2-Theta (°) exp.** | **d (Å) exp.** | **Intensity (%)** | **2-Theta (°) calc.** |
|---|---|---|---|
| 9.84 | 8.979 | 17 | 9.85 |
| 12.40 | 7.134 | 15 | 12.46 |
| 13.31 | 6.646 | 19 | 13.33 |
| 15.14 | 5.848 | 18 | 15.16 |
| 15.98 | 5.543 | 18 | 15.91 |
| 16.62 | 5.329 | 19 | 16.66 |
| 17.95 | 4.939 | 100 | 17.96 |
| 18.88 | 4.697 | 65 | 18.93 |
| 20.49 | 4.332 | 24 | 20.52 |
| 20.99 | 4.228 | 34 | 20.99 |
| 23.07 | 3.852 | 39 | 23.11 |
| 23.44 | 3.792 | 36 | 23.48 |
| 24.28 | 3.663 | 58 | 24.27 |
| 25.10 | 3.545 | 19 | 25.18 |
| 26.02 | 3.422 | 15 | 26.02 |
| 26.82 | 3.322 | 19 | 26.85 |
| 27.51 | 3.239 | 16 | 27.56 |

3. Process for the preparation of the crystalline form V of the compound of formula (I) according to claim 1, **characterised in that** the solvents used are ethanol and isopropyl ether.

4. Process for the preparation of the crystalline form V of the compound of formula (I) according to claim 1, **characterised in that** the gas used is nitrogen.

5. Process for the preparation of the crystalline form V of the compound of formula (I) according to claim 1, **characterised in that** the minimum concentration of the agomelatine solution used is 5 g/l.

## Patentansprüche

1. Verfahren zur Herstellung der Kristallform V von Agomelatin der Formel (I): 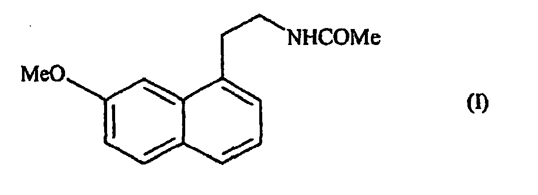 **dadurch gekennzeichnet, dass** man eine Lösung von Agomelatin in einem oder zwei in beliebigen Verhältnissen mischbaren Lösungsmitteln, deren Siedetemperatur unterhalb 120 °C liegt, in einem Sprühtrockner zerstäubt.

2. Verfahren zur Herstellung der Kristallform V der Verbindung der Formel (I) nach Anspruch 1, **dadurch gekennzeichnet, dass** die erhaltene Verbindung das folgende Pulverröntgenbeugungsdiagramm, gemessen mit einem Siemens Diffraktometer D5005 (Kupfer-Antikathode) und ausgedrückt als Netzebenenabstände d, Bragg-2-Theta-Winkeln und relative Intensitäten (ausgedrückt als Prozentsatz bezogen auf die intensivste Bande) aufweist:
| **2-Theta (°) gemessen** | **d (Å) gemessen** | **Intensität (%)** | **2-Theta (°) berechnet** |
|---|---|---|---|
| 9,84 | 8,979 | 17 | 9,85 |
| 12,40 | 7,134 | 15 | 12,46 |
| 13,31 | 6,646 | 19 | 13,33 |
| 15,14 | 5,848 | 18 | 15,16 |
| 15,98 | 5,543 | 18 | 15,91 |
| 16,62 | 5,329 | 19 | 16,66 |
| 17,95 | 4,939 | 100 | 17,96 |
| 18,88 | 4,697 | 65 | 18,93 |
| 20,49 | 4,332 | 24 | 20,52 |
| 20,99 | 4,228 | 34 | 20,99 |
| 23,07 | 3,852 | 39 | 23,11 |
| 23,44 | 3,792 | 36 | 23,48 |
| 24,28 | 3,663 | 58 | 24,27 |
| 25,10 | 3,545 | 19 | 25,18 |
| 26,02 | 3,422 | 15 | 26,02 |
| 26,82 | 3,322 | 19 | 26,85 |
| 27,51 | 3,239 | 16 | 27,56 |

3. Verfahren zur Herstellung der Kristallform V der Verbindung der Formel (I) nach Anspruch 1, **dadurch gekennzeichnet, dass** man als Lösungsmittel Ethanol und Isopropylether verwendet.

4. Verfahren zur Herstellung der Kristallform V der Verbindung der Formel (I) nach Anspruch 1, **dadurch gekennzeichnet, dass** man als Gas Stickstoff verwendet.

5. Verfahren zur Herstellung der Kristallform V der Verbindung der Formel (I) nach Anspruch 1, **dadurch gekennzeichnet, dass** die Konzentration der verwendeten Lösung von Agomelatin mindestens 5 g/l beträgt.
